# EUROPÄISCHE PATENTSCHRIFT

(11) **EP 0 344 419 B1**
(45) Veröffentlichungstag und Bekanntmachung des Hinweises auf die Patenterteilung: **27.01.1993**
(21) Anmeldenummer: 89104882.9
(22) Anmeldetag: 18.03.1989
(51) Int. Cl.: C07C 69/33, C07C 69/52, C07C 69/732, C07C 67/03, C11C 3/02, C07C 67/02

(54) **Verfahren zur Herstellung von Fettsäure- oder Hydroxyfettsäureestern von Isopropylidenderivaten eines Polyglycerins**
Method for preparing fatty acid or hydroxy-fatty acid esters of isopropylidene derivatives of a polyglycerol
Procédé pour la fabrication d'esters d'acides gras ou d'hydroxy-acides gras de dérivés isopropylidènes d'un polyglycérol

(30) Priorität: 30.05.1988 DE 3818292
(43) Veröffentlichungstag der Anmeldung: 06.12.1989
(73) Patentinhaber: DEUTSCHE SOLVAY-WERKE GMBH, 42697 Solingen (DE)
(72) Erfinder: Jakobson, Gerald, Dr. Dipl.-Chem., D-4134 Rheinberg 2 (DE); Siemanowski, Werner, Dr. Dipl.-Chem., D-4134 Rheinberg 1 (DE); Uhlig, Karl-Heinz, D-4150 Krefeld-Traar (DE)
(74) Vertreter: Seiler, Siegfried

(56) Entgegenhaltungen:
- EP-A- 0 291 870
- FETTE, SEIFEN, ANSTRICHMITTEL, Band 62, Heft 9, 1960, Seiten 796-802, Berlin-Hamburg; H.P. KAUFMANN et al.: "Ueber Fettsäureester des Diglycerins"

## Beschreibung

Die vorliegende Erfindung betrifft ein Verfahren zur Herstellung von Fettsäure- oder Hydroxyfettsäureester von Isopropylidenderivaten eines Polyglycerins aus Fettsäurealkylestern mit C₆ - C₂₂ in der Fettsäurekomponente und C₁ - C₄ in der Esterkomponente, die im alkalischen Medium mit einem oder mehreren Isopropylidenderivaten mindestens eines Polyglycerins umgesetzt werden, wobei bestimmte Verfahrensbedingungen eingehalten werden.

Es ist bereits bekannt, Fettsäureester der Polyglycerine durch mehrtägiges Erhitzen von Diglycerin mit einem großen Überschuß von Fettsäuren, z. B. als Tetraester von Laurin-, Palmitin-, Stearin- und Ölsäure in Form brauner, fester oder öliger Verbindungen herzustellen ((vgl. C.A. 41,2392 (1947)). Infolge der langen Erhitzungsdauer können nur stark verunreinigte Produkte mit sehr schlechten Ausbeute erhalten werden. Man hat daher versucht, entsprechende Verbindungen aus Isopropyliden-Diglycerin mit Stearylchlorid in Chloroform zu erhalten. Jedoch ist der dabei erforderliche Arbeitsaufwand beträchtlich, da nach der Umsetzung das Gemisch zu einem Brei erstarrt, das nach 36 Std. mit Benzol aufgenommen werden muß. Nach Abtrennung der wäßrigen Lösung und Waschen mit Wasser, die überschüssige Stearinsäure durch Schütteln mit 10%iger Natriumbicarbonatlösung entfernt, über Natriumsulfat getrocknet und das Lösungsmittel abdestilliert werden muß. Der dabei entstehende Rückstand muß zwei- oder mehrfach mit Alkoholen umkristallisiert werden. Nachteilig bei diesem Verfahren ist weiterhin, daß Hydrochloride in molaren Verhältnissen anfallen, die entweder weiter verarbeitet werden müssen oder umweltbelastende Stoffe darstellen (siehe Dokument Fette ,Seifen ,Anstrichmittel, Band 62, Heft 9, 1960, Seiten.796-802, Berlin -Hamburg; H.P.Kaufman et al).

Ziel und Aufgabe der vorliegenden Erfindung war es daher ein verbessertes Verfahren zur Herstellung von Fettsäurealkylestern von Mono- oder Diisopropylidenderivaten des Polyglycerins zu finden. Insbesondere sollte es ermöglicht werden, definiert Fettsäurealkylester von Mono- oder Diisopropylidenderivaten des Polyglycerins in einer hohen Ausbeute zu erreichen.

Erfindungsgemäß wurde festgestellt, daß diesen Zielen und Aufgaben ein Verfahren zur Herstellung von Fettsäure- oder Hydroxyfettsäureestern von Isopropylidenderivaten eines Polyglycerins gerecht wird, durch Umsetzung von Fettsäurealkylestern, Mono- oder Polyhydroxyfettsäurealkylestern mit C₆ - C₂₂ in der Fettsäurekomponente und C₁ - C₄ in der Esterkomponente, im alkalischen Medium mit einem oder mehreren Isopropylidenderivaten eines Polyglycerins.

Gemäß der Erfindung wird die Umsetzung bei Temperaturen von 140 - 220 °C, vorzugsweise 170 - 200 °C, und im Vakuum bei 950 - 5 mbar, vorzugsweise bei 500 - 10 mbar, durchgeführt, der dabei entstehende C₁ - C₄ Alkohol durch Destillation entfernt, vorzugsweise kontinuierlich entfernt und das Reaktionsprodukt gereinigt, vorzugsweise durch Filtration, Zentrifugation, Destillation und/oder fraktionierte Destillation gereinigt.

Wird die Reaktion bei unter 150 °C durchgeführt, so treten keine ausreichenden Reaktionsgeschwindigkeiten auf, während bei Temperaturen von über 220 °C ein hoher Prozentsatz unerwünschter Nebenprodukte anfällt. Führt man andererseits die Reaktion bei Normaldruck durch, tritt eine längere Umsetzungsdauer auf und ebenfalls ein höherer Prozentsatz unerwünschter Nebenprodukte. Als Fettsäurealkylester werden gesättigte oder ungesättigte, verzweigte oder unverzweigte Fettsäurealkylester eingesetzt, u.a. Ester von Vorlauffettsäuren C₆-C₁₀, Laurinsäure, Myristinsäure, Cocosfettsäure, Stearinsäure, Behensäure und/oder 2- Ethylhexansäure, Isostearinsäure, Palmölfettsäure, Ölfettsäure, Sojaölfettsäure und/oder Linolsäure.

Als Hydroxyfettsäurealkylester werden u.a. 12-Hydroxystearinsäureäthylester und/oder Rizinolfettsäureester eingesetzt.

Als alkalisch reagierende Verbindung zur Beschleunigung der Reaktion wird bevorzugt mindestens ein Alkali- und /oder Erdalkalihydroxid, -bicarbonat, und/oder -carbonat und/oder -alkoholat und/oder eine Alkali- und /oder Erdalkaliseife zugefügt.

Nach einer bevorzugten Ausführungsform wird die Umsetzung der Fettsäurealkylester, Mono- oder Polyhydroxyfettsäurealkylester mit einem oder mehreren Isopropylidenderivaten eines Polyglycerins in Gegenwart von weniger als 5 Gew.-% Wasser, vorzugsweise weniger als 1 Gew.-% Wasser (bezogen auf die Gesamtmenge der umzusetzenden Verbindungen) durchgeführt. Gemäß dieser Ausführungsform können gewisse Nebenreaktionen vermindert werden, so daß der Gehalt an evtl. Verunreinigungen reduziert werden kann.

Als Isopropylidenderivate des Polyglycerins werden bevorzugt Mono- und/oder Diisopropylidenderivate des Di-, Tri- und/oder Tetraglycerins (ausgenommen Diisopropylidendiglycerin) eingesetzt.

Nach einer bevorzugten Ausführungsform des erfindungsgemäßen Verfahrens erfolgt die Umsetzung des Fettsäurealkylesters, Mono- und/oder Polyhydroxyfettsäurealkylesters mit einem Mono- und/oder Diisopropylidenderivat eines Di- (ausgenommen Diisopropylidendiglycerin), Tri- und/oder Tetraglycerins pro umzusetzende Hydroxylgruppe (bezogen auf die Mono- oder Diisopropylidenderivate des jeweiligen Polyglycerins) in einem 1,2 bis 3-fachen, vorzugsweise 1,5- bis 2,5-fachen, molaren Überschuß an Fettsäurealkylester. Durch diesen Überschuß gelingt es eine höhere Ausbeute zu erhalten.

Nach einer bevorzugten Ausführungsform wird im Falle der Umsetzung eines Mono- und/oder Diisopropylidenderivates des Di- und/oder Tetraglycerins (ausgenommen Diisopropylidendiglycerin) mit Fettsäurealkylestern, Mono- oder Polyhydroxyfettsäurealkylstern ein 2,1 - 10-facher, vorzugsweise 4- bis 8-facher, molarer Überschuß an Monoisopropylidendiglycerin und/oder Diisopropylidentetraglycerin (bezogen auf Fettsäurealkylester, Mono- oder Polyhydroxyfettsäurealkylester) eingesetzt. Dadurch wird der Gehalt an gebildeten Monoestern stark erhöht.

Nach einer bevorzugten Ausführungsform werden nach der Umsetzung des Fettsäurealkylesters, Mono- oder Polyhydroxyfettsäurealkylesters anfallende Salze bei 20 - 120 °C, vorzugsweise 40 - 80 °C, in an sich bekannter Weise durch Filtration abgetrennt.

Nach einer weiteren bevorzugten Ausführungsform wird nach der Umsetzung des Fettsäurealkylesters, Monooder Polyhydroxyfettsäurealkylesters mit einem oder mehreren Iso- bzw. Diisopropylidenderivaten des Di-, Tri- und/oder Tetraglycerins (ausgenommen Diisopropylidendiglycerin) der Überschuß des nicht umgesetzten Fettsäurealkylesters, Mono- oder Polyhydroxyfettsäurealkylesters und/oder der nichtumgesetzten Iso- bzw. Diisopropylidenderivate des Di-, Tri- und/oder Tetraglycerins im Vakuum abdestilliert und das zurückbleibende Umsetzungsprodukt in an sich bekannter Weise gegebenenfalls destillativ aufgetrennt.

Die Erfindung betrifft weiterhin die Verwendung der erfindungsgemäß hergestellten Fettsäure- oder Hydroxyfettsäureester der Mono- und/oder Diisopropylidenderivate des Di-, Tri- und/oder Tetraglycerins als Zwischenprodukte für die Herstellung von nichtionogenen Tensiden.

Die Fettsäure- oder Hydroxyfettsäureester (einschließlich Polyhydroxyfettsäureester) der Mono- und/oder Diisopropylidenderivate des Di-, Tri- und/oder Tetraglycerins (ausgenommen Diisopropylidendiglycerin) werden erfindungsgemäß als Lösemittel und/oder Lösungsvermittler für lipidlösliche oder öllösliche Wirkstoffe, vorzugsweise Biozide, pharmazeutische Wirkstoffe, holzkonservierende Mittel, Farben und Anstrichmittel und/oder für kosmetische Zubereitungen oder Hautpflegemittel verwendet.

### Ausführungsbeispiele

### 1. Umsetzung von Fettsäurealkylestern mit Diisopropylidentriglycerin

1,52 kg (5 mol) Ölsäuremethylester und 25 g (0,2 mol) Kaliumcarbonat werden in einen 4 l-Kolben gegeben und unter Rühren auf ca. 160 °C aufgeheizt. Bei ca. 100 mbar Unterdruck werden evtl. vorhandene geringe Mengen an Wasser abdestilliert. Daraufhin werden 820 g (2,5 mol) Diisopropylidentriglycerin zugegeben und die Reaktionstemperatur auf 180 °C - 190 °C erhöht. Bei 400 - 50 mbar wird das entstehende Methanol destillativ entfernt. Nach 4 - 5 stündiger Reaktionszeit wird der Ansatz auf ca. 70 °C abgekühlt und ausgefallene Bestandteile abfiltriert.

Überschüssiger Fettsäurealkylester sowie nicht umgesetztes Diisopropylidentriglycerin werden bei ≦ 0,4 mbar und ca. 160 °C Kopftemperatur destillativ entfernt. Das verbleibende Rohprodukt wird anschließend in einer Kurzwegverdampferanlage bei ≦ 0,1 mbar und 210 °C Ölvorlauftemperatur feindestilliert.

### 2. Umsetzung von Fettsäurealkylestern mit Monoisopropylidendiglycerin

300 g (ca. 1 mol) Palmitinsäuremethylester und 0,5 g Lithiumhydroxid ·1 H₂O werden in einen 2 l-Kolben gegeben und unter Rühren auf 160 °C erwärmt. Bei 150 mbar wird das (aus dem Katalysator sowie mit dem Fettsäureester eingeschleppten) Wasser destillativ entfernt.

Daraufhin werden 1,031 kg (5 mol) Monoisopropylidendiglycerin zugegeben und die Reaktionstemperatur auf 190 °C erhöht. Bei 400 - 50 mbar (geringer Druck zum Ende der Reaktion) wird das entstehende Methanol destillativ entfernt. Nach 3-stündiger Reaktionszeit wird der Ansatz auf 45 °C abgekühlt und ausgefallene Bestandteile abfiltriert.

Überschüssiges Monoisopropylidendiglycerin wird bei ≦ 0,2 mbar und ca. 140 °C Ölvorlauftemperatur in einer Kurzwegverdampferanlage destillativ entfernt, das verbleibende Rohprodukt in der gleichen Anlage bei ≦ 0,2 mbar und 205 °C Ölvorlauftemperatur feindestilliert.

## Patentansprüche

1. Verfahren zur Herstellung von Fettsäure- oder Hydroxyfettsäureestern von Isopropylidenderivaten eines Polyglycerins aus Fettsäurederivaten, die mit einem oder mehreren Isopropylidenderivaten eines Polyglycerins umgesetzt werden, dadurch gekennzeichnet, daß als Fettsäurederivate Fettsäurealkylester, Mono- oder Polyhydroxyfettsäurealkylester mit C₆ - C₂₂ in der Fettsäurekomponente und C₁ - C₄ in der Esterkomponente verwendet und mit Isopropylidenderivaten eines Polyglycerins im alkalischen. Medium umgesetzt werden, daß die Umsetzung bei Temperaturen von
140 - 220 °C, vorzugsweise
170 - 200 °C,
und im Vakuum
bei 950 - 5 mbar, vorzugsweise
bei 500 - 10 mbar,
durchgeführt und der dabei entstehende C₁ - C₄ Alkohol durch Destillation entfernt, vorzugsweise kontinuierlich entfernt und das Reaktionsprodukt gereinigt, vorzugsweise durch Filtration, Zentrifugation, Destillation und/oder fraktionierte Destillation gereinigt wird.

2. Verfahren nach Anspruch 1, dadurch gekennzeichnet, daß als alkalisch reagierende Verbindung zur Beschleunigung der Reaktion mindestens ein Alkali- und /oder Erdalkalihydroxid, -bicarbonat, und/oder -carbonat und/oder -alkoholat und/oder eine Alkali- und /oder Erdalkaliseife zugefügt wird.

3. Verfahren nach Ansprüchen 1 und 2, dadurch gekennzeichnet, daß die Umsetzung in Gegenwart von weniger als 5 Gew.-% Wasser, vorzugsweise weniger als 1 Gew.-% Wasser (bezogen auf die Gesamtmenge der umzusetzenden Verbindungen) durchgeführt wird.

4. Verfahren nach einem oder mehreren der Ansprüche 1 bis 3, dadurch gekennzeichnet, daß als Isopropylidenderivate des Polyglycerins Mono- und/oder Diisopropylidenderivate (ausgenommen Diisopropylidendiglycerin) des Di-, Tri- und/oder Tetraglycerins eingesetzt werden.

5. Verfahren nach einem oder mehreren der Ansprüche 1 bis 4, dadurch gekennzeichnet, daß die Umsetzung des Fettsäurealkylesters, Mono- oder Polyhydroxyfettsäurealkylesters mit einem Mono- und/oder Diisopropylidenderivat eines Di-(ausgenommen Diisopropylidendiglycerin), Tri- und/oder Tetraglycerins pro umzusetzende Hydroxylgruppe (bezogen auf die Mono- oder Diisopropylidenderivate des jeweiligen Polyglycerins) in einem 1,2- bis 3-fachen, vorzugsweise 1,5-bis 2,5-fachen, Überschuß an Fettsäurealkylester, Mono- oder Polyhydroxyfettsäurealkylester erfolgt.

6. Verfahren nach einem oder mehreren der Ansprüche 1 bis 5, dadurch gekennzeichnet, daß im Falle der Umsetzung eines Mono- und/oder Diisopropylidenderivates des Di-, Tri- und/oder Tetraglycerins (ausgenommen Diisopropylidendiglycerin) mit Fettsäurealkylestern, Mono- oder Polyhydroxyfettsäurealkylestern ein 2,1- bis 10-facher, vorzugsweise 4- bis 8-facher, molarer Überschuß an Monoisopropyliden diglycerin und/oder Diisopropylidentriglycerin und/oder Diisopropylidentetraglycerin, bezogen auf Fettsäurealkylester, Mono- oder Polyhydroxyfettsäurealkylestern eingesetzt wird.

7. Verfahren nach einem oder mehreren der Ansprüche 1 bis 6, dadurch gekennzeichnet, daß nach der Umsetzung des Fettsäurealkylesters, Mono- oder Polyhydroxyfettsäurealkylesters anfallende Salze bei 20 bis 120 °C, vorzugsweise 40 bis 80 °C, in an sich bekannter Weise durch Filtration abgetrennt werden.

8. Verfahren nach einem oder mehreren der Ansprüche 1 bis 5, dadurch gekennzeichnet, daß nach der Umsetzung des Fettsäurealkylesters, Mono- oder Polyhydroxyfettsäurealkylesters mit einem oder mehreren Isopropylidenderivaten des Di-, Tri- und/oder Tetraglycerins der Überschuß des nicht umgesetzten Fettsäurealkylesters, Mono- oder Polyhydroxyfettsäurealkylesters und/oder des nichtumgesetzten Isopropylidenderivates des Diglycerins und/oder Diisopropylidenderivates des Triglycerins und Tetraglycerins im Vakuum abdestilliert und das zurückbleibende Umsetzungsprodukt in an sich bekannter Weise gegebenenfalls destillativ aufgetrennt wird.

9. Verwendung der nach den Ansprüchen 1 bis 8 hergestellten Fettsäure- oder Hydroxyfettsäureester von Mono- und/oder Diisopropylidenderivaten des Polyglycerins als Zwischenprodukte für die Herstellung von nichtionogenen Tensiden.

10. Verwendung der nach den Ansprüchen 1 bis 8 hergestellten Fettsäure- oder Hydroxyfettsäureester von Mono- und/oder Diisopropylidenderivaten des Polyglycerins als Lösemittel und/oder Lösungsvermittler für lipidlösliche oder öllösliche Wirkstoffe, vorzugsweise Biozide, pharmazeutische Wirkstoffe, holzkonservierende Mittel, Farben und Anstrichmittel und/oder für kosmetische Zubereitungen oder Hautpflegemittel.

## Claims

1. A method for producing fatty acid or hydroxyfatty acid esters of isopropylidene derivatives of a polyglycerol from fatty acid derivatives which are reacted with one or more isopropylidene derivatives of a polyglycerol, characterised in that fatty acid alkyl esters, monohydroxy or polyhydroxy-fatty acid alkyl esters with C₆ - C₂₂ in the fatty acid constituent and C₁ - C₄ in the ester component are used as fatty acid derivatives and are reacted with isopropylidene derivatives of a polyglycerol in an alkaline medium, that the reaction is carried out at temperatures of
140 - 220°C, preferably
170 - 200°C,
and in a vacuum
at 950 - 5 mbar, preferably
at 500 - 10 mbar,
and the resulting C₁ - C₄ alcohol is removed, preferably continuously removed, by distillation, and the reaction product is purified, preferably by filtration, centrifugation, distillation and/or fractional distillation.

2. A method according to Claim 1, characterised in that at least one alkali and/or alkaline earth hydroxide, bicarbonate and/or carbonate and/or alcoholate and/or an alkali and/or alkaline earth soap is added as the alkaline-reacting compound to accelerate the reaction.

3. A method according to Claims 1 and 2, characterised in that the reaction is carried out in the presence of less than 5% by weight water, preferably less than 1% by weight water (relative to the total quantity of the compounds being reacted).

4. A method according to one or more of Claims 1 to 3, characterised in that monoisopropylidene and/or diisopropylidene derivatives (with the exception of diisopropylidene diglycerol) of diglycerol, triglycerol and/or tetraglycerol are used as isopropylidene derivatives of the polyglycerol.

5. A method according to one or more of Claims 1 to 4, characterised in that the reaction of the fatty acid alkyl ester, monohydroxy or polyhydroxy-fatty acid alkyl ester with a monoisopropylidene and/or diisopropylidene derivative of a diglycerol (with the exception of diisopropylidene diglycerol), triglycerol and/or tetraglycerol per hydroxyl group being reacted (relative to the monoisopropylidene or diisopropylidene derivatives of the respective polyglycerol) takes place in a 1.2 to 3 times, preferably 1.5 to 2.5 times, excess of fatty acid alkyl ester, monohydroxy or polyhydroxyfatty acid alkyl ester.

6. A method according to one or more of Claims 1 to 5, characterised in that in the case of the reaction of a monoisopropylidene and/or diisopropylidene derivative of diglycerol, triglycerol and/or tetraglycerol (with the exception of diisopropylidene diglycerol) with fatty acid alkyl esters, monohydroxy or polyhydroxy-fatty acid alkyl esters, a 2.1 to 10 times, preferably 4 to 8 times, molar excess of monoisopropylidene diglycerol and/or diisopropylidene triglycerol and/or diisopropylidene tetraglycerol relative to fatty acid alkyl ester, monohydroxy or polyhydroxy-fatty acid alkyl esters is used.

7. A method according to one or more of Claims 1 to 6, characterised in that salts produced after the reaction of the fatty acid alkyl ester, monohydroxy or polyhydroxy-fatty acid alkyl ester are separated off at 20 to 120°C, preferably 40 to 80°C, in known manner by filtration.

8. A method according to one or more of Claims 1 to 5, characterised in that after the reaction of the fatty acid alkyl ester, monohydroxy or polyhydroxy-fatty acid alkyl ester with one or more isopropylidene derivatives of diglycerol, triglycerol and/or tetraglycerol the excess of the non-reacted fatty acid alkyl ester, monohydroxy or polyhydroxy-fatty acid alkyl ester and/or the non-reacted isopropylidene derivative of diglycerol and/or diisopropylidene derivative of triglycerol and of tetraglycerol is distilled off in a vacuum and the reaction product remaining is separated in known manner, optionally by distillation.

9. The use of the fatty acid esters or hydroxy-fatty acid esters of monoisopropylidene and/or diisopropylidene derivatives of polyglycerol produced according to Claims 1 to 8 as intermediate products for the production of non-ionogenic surfactants.

10. The use of the fatty acid esters or hydroxy-fatty acid esters of monoisopropylidene and/or diisopropylidene derivatives of polyglycerol produced according to Claims 1 to 8 as solvents and/or solubilisers for lipid-soluble or oil-soluble active substances, preferably biocides, pharmaceutical active substances, wood preservation agents, paints and painting materials and/or for cosmetic preparations or skin-care agents.

## Revendications

1. Procédé de préparation d'esters d'acides gras ou d'esters d'acides gras hydroxylés de dérivés d'isopropylidène d'une polyglycérine à partir de dérivés d'acides gras que l'on fait réagir avec un ou plusieurs dérivés d'isopropylidène d'une polyglycérine, caractérisé en ce que l'on utilise comme dérivés d'acides gras des alkyl esters d'acides gras ou des alkyl esters d'acides gras mono ou polyhydroxylés en C₆-C₂₂ dans le composant acide gras et en C₁-C₄ dans le composant ester, que l'on fait réagir en milieu alcalin avec des dérivés d'isopropylidène d'une polyglycérine, que la réaction s'effectue à une température de 140 à 220 °C, de préférence 170 à 200 °C, et sous un vide de 950-5 mbars, de préférence 500-10 mbars, que l'alcool en C₁-C₄ ainsi obtenu est éliminé par distillation, de préférence éliminé en continu, et que le produit réactionnel est nettoyé, de préférence, par filtration, centrifugation, distillation et/ou distillation fractionnée.

2. Procédé selon la revendication 1, caractérisé en ce que l'on ajoute comme composé à réaction alcaline permettant d'accélérer la réaction au moins un hydroxyde, un bicarbonate et/ou un carbonate et/ou un alcoolate de métal alcalin et/ou de métal alcalino-terreux et/ou un savon de métal alcalin et/ou de métal alcalino-terreux.

3. Procédé selon la revendication 1 ou 2, caractérisé en ce que la réaction se fait en présence de moins de 5 % en poids d'eau, de préférence moins de 1 % en poids d'eau (par rapport à la quantité totale des composés réactionnels).

4. Procédé selon une ou plusieurs des revendications 1 à 3, caractérisé en ce que l'on utilise comme dérivés d'isopropylidène de la polyglycérine des dérivés de mono et/ou diisopropylidène de la di, de la tri et/ou de la tétraglycérine (à l'exception de la diisopropylidènediglycérine).

5. Procédé selon une ou plusieurs des revendications 1 à 4, caractérisé en ce que la réaction de l'alkyl ester d'acides gras ou de l'alkyl ester d'acides gras mono et/ou polyhydroxylés avec un dérivé de mono et/ou diisopropylidène d'une di (à l'exception de la diisopropylidènediglycérine), tri et/ou tétraglycérine est réalisée avec un excès molaire de 1,2 à 3 fois, de préférence 1,5 à 2,5 fois, d'alkyl ester d'acides gras par groupe hydroxylé à convertir (par rapport aux dérivés de mono ou diisopropylidène de la polyglycérine respective).

6. Procédé selon une ou plusieurs des revendications 1 à 5, caractérisé en ce que, dans le cas de la réaction d'un dérivé de mono et/ou diisopropylidène de la di, tri et/ou tétraglycérine (à l'exception de la diisopropylidènediglycérine) avec des alkyl esters d'acides gras ou des alkyl esters d'acides gras mono ou polyhydroxylés, on utilise un excès molaire de 2,1 à 10 fois, de préférence 4 à 8 fois, de monoisopropylidènediglycérine et/ou de diisopropylidènetriglycérine et/ou de diisopropylidènetétraglycérine par rapport à l'alkyl ester d'acides gras ou l'alkyl ester d'acides gras mono ou polyhydroxylés.

7. Procédé selon une ou plusieurs des revendications 1 à 6, caractérisé en ce que, après la réaction de l'alkyl ester d'acides gras ou de l'alkyl ester d'acides gras mono ou polyhydroxylés, les sels obtenus sont séparés de manière connue en soi par filtration à 20-120 °C, de préférence à 40-80 °C.

8. Procédé selon une ou plusieurs des revendications 1 à 5, caractérisé en ce que, après la réaction de l'alkyl ester d'acides gras ou de l'alkyl ester d'acides gras mono ou polyhydroxylés avec un ou plusieurs dérivés d'iso- ou de diisopropylidène de la di, tri et/ou tétraglycérine (à l'exception de la diisopropylidènediglycérine), l'excès d'alkyl ester d'acides gras ou d'alkyl ester d'acides gras mono ou polyhydroxylés qui n'a pas réagi et/ou du dérivé d'iso- ou de diisopropylidène de la di, tri et/ou tétraglycérine qui n'a pas réagi sont séparés par distillation sous vide et le produit réactionnel subsistant est séparé éventuellement par distillation de manière connue en soi.

9. Emploi des esters d'acides gras ou d'acides gras hydroxylés des dérivés de mono et/ou diisopropylidène de la polyglycérine préparés selon les revendications 1 à 8 à titre de produits intermédiaires pour la préparation d'agents tensioactifs non ionogènes.

10. Emploi des esters d'acides gras ou d'acides gras hydroxylés de mono et/ou de diisopropylidène de la polyglycérine préparés selon les revendications 1 à 8 comme solvants et/ou médiateurs de dissolution pour les principes actifs solubles dans les lipides ou dans les huiles, de préférence les biocides, les principes actifs pharmaceutiques, les agents de conservation du bois, les colorants et les peintures et/ou pour les préparations cosmétiques ou les agents de traitement de la peau.
